# EUROPEAN PATENT SPECIFICATION

(11) **EP 2 867 199 B1**
(45) Date of publication and mention of the grant of the patent: **18.04.2018**
(21) Application number: 13813669.2
(22) Date of filing: 27.06.2013
(51) Int. Cl.: A61K 9/20, A61K 47/12, A61K 47/14, A61K 47/36, A61K 47/38, A61K 31/195, A61K 31/122, A61K 31/222

(54) **STABLE COMPOSITIONS OF FESOTERODINE**
STABILE ZUSAMMENSETZUNGEN AUS FESOTERODIN
COMPOSITIONS STABLES DE FÉSOTÉRODINE

(30) Priority: 02.07.2012 IN CH26332012
(43) Date of publication of application: 06.05.2015
(73) Proprietor: Hetero Research Foundation, Hyderabad 500 018, Telangana (IN)
(72) Inventor: PARTHASARADHI REDDY, Bandi, Hyderabad Telangana 500 018 (IN); KHADGAPATHI, Podili, Hyderabad Telangana 500 018 (IN); SYAMPRASAD, Borra, Hyderabad Telangana 500 055 (IN)
(74) Representative: ZBM Patents ApS
(86) International application number: PCT/IN2013/000396
(87) International publication number: WO 2014/006636

(56) References cited:
- EP-A1- 2 508 173
- EP-A1- 2 508 175
- WO-A1-2007/141298
- WO-A1-2011/117884
- US-A1- 2010 130 606

## Description

### PRIORITY

This patent application claims priority to Indian patent application number 2633/CHE/2012, filed on July 2, 2012.

### FIELD OF THE INVENTION

The present invention encompasses stable pharmaceutical compositions of fesoterodine or pharmaceutically acceptable salts thereof.

### BACKGROUND OF THE INVENTION

Chemically, fesoterodine fumarate is designated as isobutyric acid 2-((R)-3-diisopropylammonium-1phenylpropyl)-4-(hydroxymethyl) phenyl ester hydrogen fumarate. Its empirical formula is C₃₀H₄₁NO₇, corresponding to a molecular weight of 527.66 having the following structural formula: 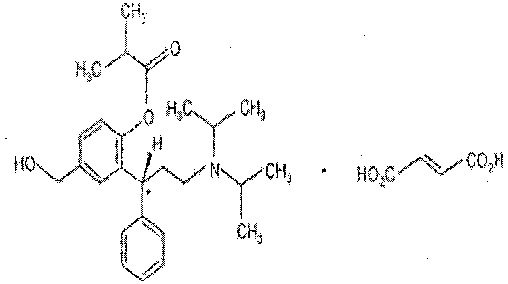 The asterisk (*) indicates the chiral carbon

Fesoterodine is marketed under the trade name TOVIAZ® in United States by Pfizer in the form of 4mg and 8mg tablets for the treatment of overactive bladder with symptoms of urge urinary incontinence, urgency, and frequency.

US6858650 and US7384980 disclose fesoterodine.

US7807715 and US8088398 disclose composition of fesoterodine comprising sorbitol or xylitol, lactose monohydrate, microcrystalline cellulose, hydroxypropyl methylcellulose, glycerol dibehenate and talc.

US20090285891 disclose controlled release composition with at least two diffusion layers and a gastro-resistant coating layer meant for intestinal release.

WO2011117884 claims composition of fesoterodine fumarate with polyvinylpyrrolidone, microcrystalline cellulose and hydroxypropyl methycelllulose.

During storage, fesoterodine compositions yields various impurities in variable quantities, which is not desirable if they are beyond certain limits.

Thus there remains a need to formulate storage stable pharmaceutical compositions of fesoterodine that contain reduced amounts of total impurities.

### SUMMARY OF THE INVENTION

The present invention discloses storage stable pharmaceutical compositions of fesoterodine or a pharmaceutically acceptable salt thereof.

One embodiment of the present invention provides stable pharmaceutical composition comprising fesoterodine fumarate, glyceryl behenate and a stabilizer.

Another embodiment of the present invention provides stable pharmaceutical compositions comprising fesoterodine fumarate in an amount of 1% to 5% by weight, glyceryl behenate in an amount of 1% to 8% by weight, pregelatinized starch in an amount of 30% to 50% by weight, and a stabilizer in an amount of 0.1% to 10% by weight, based on total weight of the composition.

In a specific embodiment, the pharmaceutical tablet compositions of fesoterodine fumarate have reduced levels of 2-((R) -3- (diisopropylamino)-1-phenylpropyl)-4-(hydroxymethyl)phenol impurity when produced and stored for certain period of time.

In another embodiment, the present invention provides a storage stable pharmaceutical composition comprising fesoterodine, having a 2-((R) -3- (diisopropylamino)-1-phenylpropyl)-4-(hydroxymethyl)phenol impurity in an amount of less than 2% by weight after storage for one month at 40°C and 75%RH.

In another embodiment, the present invention provides a storage stable controlled release tablet composition comprising fesoterodine fumarate as an active agent; glyceryl behenate as a lubricant; pregelatinized starch as a diluent; hydroxypropyl methylcellulose as controlled-release agent; and as a mixture of citric acid and pregelatinized starch. In another embodiment, the present invention provides a process for the preparation of pharmaceutical tablet composition of fesoterodine comprising: (i) sifting and blending one or more pharmaceutically acceptable excipients with glyceryl behenate to form a dry mixture; (ii) sifting and blending dry mixture of step (i) with fesoterodine; (iii) optionally lubricating the blend of step (ii); and (iv) directly compressing the blend of step (ii) or (iii) into tablets.

In yet another embodiment, the pharmaceutical composition comprising a therapeutically effective amount of fesoterodine is useful for the treatment of overactive bladder with symptoms of urge urinary incontinence, urgency, and frequency.

### DETAILED DESCRIPTION OF THE INVENTION

The present invention discloses storage stable pharmaceutical compositions of fesoterodine or a pharmaceutically acceptable salt thereof.

The term "fesoterodine" as used herein includes fesoterodine in the form of a free base or a pharmaceutically acceptable salt which includes the fumarate, hydrochloride, hydrobromide, nitrate, sulfate, mandelate, oxalate, succinate, maleate, besylatetosylate, palmitate and tartarate salts. More specifically the fesoterodine is fesoterodine fumarate.

The term "storage stable" as used herein refers to solid dosage forms of fesoterodine with reduced levels of total impurities when stored for certain period of time.

Storage stable pharmaceutical compositions of fesoterodine are important to control the levels of impurities in the final dosage form and to ensure that the impurity is present in the lowest possible levels.

The term "composition" or "pharmaceutical composition" or "formulation" or "solid oral composition" or "dosage form" as used herein synonymously include solid dosage forms such as tablets, capsules, granules, mini-tablets and the like meant for oral administration, more specifically tablets.

Compositions of the present invention can be developed into solid oral dosage forms to exhibit extended release, sustained release, controlled release, modified release and delayed release or a combination thereof, using rate controlling polymers. Preferably, the composition of the invention is in the form of an extended release composition.

The term 'extended release" used herein refer to a composition that provides release of fesoterodine or salts thereof over a period of 24 hours.

The term "effective amount" or "pharmaceutically effective amount" used interchangeably, is defined to mean the amount or quantity of the active drug (e.g.,fesoterodine), which is sufficient to elicit an appreciable biological response when administered to the patient. It will be appreciated that the precise therapeutic dose will depend on the age and condition of the patient, nature of the condition to be treated and will be at the ultimate discretion of the attendant physician.

As used in this specification and the appended claims, the singular forms "a", "an", and "the" include plural references unless the context clearly dictates otherwise. Thus for example, a reference to "a method" includes one or more methods, and/or steps of the type described herein and/or which will become apparent to those persons skilled in the art upon reading this disclosure and so forth.

The term "excipient" means a pharmacologically inactive component such as a diluent, disintegrant, carrier, and the like, of a pharmaceutical product. The excipients that are useful in preparing a pharmaceutical composition are generally safe, non-toxic and are acceptable for veterinary as well as human pharmaceutical use. Reference to an excipient includes both one excipient and more than one excipient.

The present invention provides storage stable pharmaceutical compositions of fesoterodine with one or more pharmaceutically acceptable excipients and process for their preparation.

In one aspect, the present invention provides a stable pharmaceutical composition comprising fesoterodine, having a 2-((R) -3- (diisopropylamirio)-1-phenylpropyl)-4-(hydroxymethyl)phenol impurity in an amount of less than 2% by weight after storage for one month at 40°C and 75%RH, specifically in an amount of less than 1.5% by weight after storage for one month at 40°C and 75%RH.

In particular, the pharmaceutical tablet compositions of fesoterodine fumarate have reduced levels of 2-((R) -3- (diisopropylamino)-1-phenylpropyl)-4-(hydroxymethyl)phenol impurity.

The present invention also describes selection of suitable pharmaceutically acceptable excipients and/or stabilizers to prepare the stable pharmaceutical compositions of fesoterodine fumarate.

According to the present invention stability of drug-excipient blend was evaluated based on percentage w/w of 2-((R) -3- (diisopropylamino)-1-phenylpropyl)-4-(hydroxymethyl)phenol impurity (H-FTFRC-01) and total impurities present in drug-excipient blend, initially and after storage for one month at 40°C and 75% relative humidity. The results are presented in Table 1.

**Table 1. Results of stability evaluation of drug-excipient blend**

| Drug-Excipients | Initial | | 40°C/75%RH,1month | |
|---|---|---|---|---|
| | H-FTFRC-01* | Total Impurities | H-FTFRC-01* | Total Impurities |
| Fesoterodine fumarate (FF^{#}) | 0.284 | 0.800 | 0.524 | 1.245 |
| FF-crospovidone | 0.233 | 0.641 | 0.777 | 1.638 |
| FF-hydroxypropyl cellulose | 0.309 | 0.619 | 0.804 | 1.254 |
| FF-polyethylene glycol | 0.326 | 0.723 | 1.063 | 1.878 |
| FF-colloidal silicon dioxide | 0.362 | 1.050 | 0.864 | 1.633 |
| FF-a graft copolymer of polyvinyl alcohol-polyethylene glycol | 0.425 | 0.838 | 1.011 | 1.8277 |
| FF-a mixture of citric acid and pregelatinized starch | 0.370 | 0.610 | 0.590 | 1.050 |
| FF-glyceryl behenate | 0.256 | 0.670 | 0.968 | 1.813 |
| FF-sorbitol | 0.707 | 1.271 | 1.44 | 2.807 |
| FF-lactose monohydrate | 0.562 | 1.009 | 6.195 | 11.398 |
| FF-microcrystalline cellulose | 0.410 | 1.409 | 2.835 | 5.237 |
| FF-dibasic calcium phosphate, anhydrous | 2.023 | 4.688 | 5.001 | 15.729 |
| FF-ethyl cellulose | 1.055 | 1.927 | 3.990 | 5.811 |
| FF-polyethylene oxides | 5.205 | 6.560 | 10.732 | 14.223 |
| FF-carbopol | 0.183 | 0.747 | 4.053 | 10.438 |
| FF-magnesium hydroxide | 9.306 | 14.830 | 17.918 | 41.697 |

| | | | | |
|---|---|---|---|---|
| H-FTFRC-01*: 2-((R)-3-(diisopropylamino)-1-phenylpropyl)-4-(hydroxymethyl) phenol. FF^{#}: Fesoterodine fumarate. | | | | |

As can be seen from Table 1, blends of festoterodine fumarate with crospovidone, hydroxypropyl cellulose, polyethylene glycol, a graft copolymer of polyvinyl alcohol-polyethylene glycol, a mixture of citric acid and pregelatinized starch, colloidal silicon dioxide or glyceryl behenate has shown less than about 1 % w/w of H-FTFRC-01 impurity after storage for one month at 40°C and 75% relative humidity. A blend containing lactose monohydrate, microcrystalline cellulose, dibasic calcium phosphate anhydrous, ethyl cellulose, polyethylene oxides, carbopol or magnesium hydroxide, for example, has shown greater than about 1 % w/w of H-FTFRC-01 impurity after storage for one month at 40°C and 75% relative humidity.

Based on the results presented in Table 1, and without being held to theory, it is believed that a mixture of citric acid and pregelatinized starch, colloidal silicon dioxide, crospovidone, hydroxypropyl cellulose, polyethylene glycol, a graft copolymer of polyvinyl alcohol-polyethylene glycol, and glyceryl behenate are compatible with fesoterodine fumarate.

Accordingly, the present invention provides a stable pharmaceutical composition comprising fesoterodine fumarate, glyceryl behenate and a mixture of citric acid and pre-gelatinized starch as stabilizer.

The present invention also provides a stable pharmaceutical composition comprising fesoterodine fumarate in an amount of 1% to 5% by weight, glyceryl behenate in an amount of 1% to 8% by weight, pregelatinized starch in an amount of 30% to 50% by weight, and the stabilizer in an amount of 0.1% to 10% by weight based on total weight of the composition.

As used herein, a stabilizer is an excipient that protects the fesoterodine fumarate composition from degradation. Exemplary stabilizers include, but are not limited to citric acid monohydrate, colloidal silicon dioxide, a mixture of citric acid and pregelatinized starch, glyceryl behenate, hydroxypropyl cellulose, crospovidone, polyethylene glycol and a graft copolymer of polyvinyl alcohol-polyethylene glycol or a combination thereof.

In certain embodiments, pharmaceutical compositions of the present invention further comprise a controlled release agent.

Controlled-release agents provide gradual release of fesoterodine over an extended period of time. Exemplary controlled-release agents include, but are not limited to hydroxypropyl methylcellulose, hydroxypropyl cellulose, polyacrylates, methyl acrylates, polyethylene glycols, chitosan, gums, starch derivatives, polyurethanes, galactomannans, polysaccharides, polyalcohols, glycerol palmitostearate, beeswax, glycowax, carnaubawax, hydrogenated vegetable oil, glycerol monostearate, stearyl alcohol, polyanhydrides, methyl acrylates, and the like, and combinations thereof. A specific controlled-release agent is hydroxypropyl methylcellulose. More specifically hydroxypropyl methylcellulose having a viscosity of 100 cps to 1,00,000 cps is used.

In one embodiment, a controlled-release fesoterodine formulation releases fesoterodine over a period of 24 hours.

In one embodiment, a pharmaceutical composition of the present invention is free from sugar alcohols. The term "sugar alcohols" used herein refers to polyhydric sugars and its derivatives, like mannitol, xylitol, maltitol, isomaltitol, erythritol, lactitol and sorbitol. "Free from sugar alcohols" means that the composition has no added sugar alcohols.

The compositions of fesoterodine optionally further comprise one or more pharmaceutically acceptable excipients selected from a diluent, a disintegrant, a glidant and a lubricant or a combination thereof.

Exemplary diluents include pregelatinized starch, starches, modified starches, and the like, and a combinations thereof.

Exemplary disintegrants include crospovidone, croscarmellose sodium, sodium starch glycolate, polacrillin potassium, polyvinylpyrrolidone, starches such as corn starch, potato starch, pre-gelatinized and modified starches, clays, bentonite, and the like, and combinations thereof.

Exemplary glidants include talc, colloidal silicon dioxide and other forms of silicon dioxide, and combinations thereof.

Suitable lubricants include glyceryl behenate, talc, calcium stearate, sodium stearyl fumarate, zinc stearate, stearic acid, fumaric acid, palmitic acid, and the like, and combinations thereof.

The compositions optionally include a film coat. A film coat on the tablet provides an elegant appearance, protects from moisture and further contributes to the ease with which it can be swallowed.

In one embodiment, it has further been observed by the present inventors that, fesoterodine fumarate compositions prepared by direct compression process were more stable as compared to compositions prepared by dry granulation process and/or wet granulation process.

The present invention provides a process for the preparation of pharmaceutical composition comprising fesoterodine and one or more pharmaceutically acceptable excipients, using direct compression.

In first aspect, a direct compression process comprises:
(i) sifting and blending one or more pharmaceutically acceptable excipients with glyceryl behenate to form a dry mixture;
(ii) sifting and blending dry mixture of step (i) with fesoterodine;
(iii) optionally lubricating the blend of step (ii); and
(iv) directly compressing the blend of step (ii) or (iii) into tablets.

In second aspect, a direct compression process comprises:
(i) sifting and blending one or more excipients with a first portion of glyceryl behenate to form a dry mixture;
(ii) sifting and blending a portion of dry mixture of step (i) with fesoterodine fumarate;
(iii) blending the materials of step (i) and (ii);
(iv) lubricating the blend of step (iii) with a second portion of of glyceryl behenate to form a lubricated blend; and
(v) directly compressing the lubricated blend of step (iv) into tablets.

In one embodiment of the method of forming a composition, the first and second portions of glyceryl benenate in steps (i) and (iv) are each half of the total amount of glyceryl behenate in the composition. In another embodiment, the portion of dry mixture of step (i) employed in step (ii) is 5-20%, specifically 10% of the dry mixture of step (i).

In a specific aspect, the process for preparing stable pharmaceutical compositions of fesoterodine involves pre-lubrication of excipients with glyceryl behenate to reduce the interaction between drug and excipients. This pre-lubrication results significantly reduced level of 2-((R) -3- (diisopropylamino)-1-phenylpropyl)-4-(hydroxymethyl)phenol impurity and total impurities in the final dosage form as compared to compositions prepared by a conventional blending process. The stability of dosage forms of fesoterodine fumarate can be improved based on sequence of mixing drug and excipients during process.

The pharmaceutical composition comprising therapeutically effective amount of fesoterodine or its pharmaceutically acceptable salt thereof is useful for the treatment of overactive bladder with symptoms of urge urinary incontinence, urgency, and frequency.

Certain specific aspects and embodiments of this invention are described in further detail by the examples below, which are provided only for purposes of illustration and are not intended to limit the scope of the invention in any manner.

### EXAMPLE-1

### Pre-lubrication of excipients with glyceryl behenate

### Fesoterodine tablets prepared according to the composition listed in Table 2 using the following steps:

1. Pregelatinized starch, citric acid monohydrate, hydroxypropyl methylcellulose K100 CR, hydroxypropyl methylcellulose K100M CR, hydroxypropyl methylcellulose K4M, and colloidal silicon dioxide were sifted with a half quantity of glyceryl behenate through a mesh #40 sieve and dry blended for 15 minutes.
2. 10% blend of step 1 and fesoterodine hydrogen fumarate were sifted together through a mesh #40 sieve.
3. sifted materials of step 1 and step 2 were blended for 10 minutes.
4. remaining half quantity of glyceryl behenate was sifted through a mesh #60 sieve.
5. blend of step 3 was lubricated with glyceryl behenateof step 4.
6. lubricated blend of step 5 was directly compressed into tablets.
7. tablets of step 6 were film coated using an Opadry® dispersion.

**Table 2.Fesoterodine tablet compositions prepared by direct compression process:**

| Ingredient | | | | | mg/Tablet | | | |
|---|---|---|---|---|---|---|---|---|
| Fesoterodine hydrogen | | fumarate | | | 8.00 | | | |
| Pregelatinized starch | | | | | 147.44 | | | |
| Hydroxypropyl | methylcellulose | | K 100 | CR | 72.00 | | | |
| Hydroxypropyl | methylcellulose | | K 100M | CR | 48.00 | | | |
| Hydroxypropyl | methylcellulose | | K4M CR | | 24.00 | | | |
| Citric acid monohydrate | | | | | 0.66 | | | |
| Colloidal silicon dioxide | | | | | 9.90 | | | |
| Glyceryl behenate | | | | | 10.00 | | | |
| | | | | | | | | |
| Core tablet weight | | | | | 320.00 | | | |
| | | | | | | | | |
| Film coating | | | | | | | | |
| Opadry® blue | | | | | 10.00 | | | |
| Coated tablet weight | | | | | 330.00 | | | |
| Dissolution study: | | | | | | | | |
| | | | | | | | | |
| Dissolution Medium | | : | 6.8 pH | phosphate | buffer | | | |
| Volume | | : | 900 ml | | | | | |
| Apparatus | | : | USP II | (Paddle) | | | | |
| Speed | | : | 75 RPM | | | | | |
| | | | | | | | | |
| Time in Hours | 1 | 2 | 4 | 6 | 8 | 12 | 16 | 20 |
| Example-1 Dissolution (%) | 16 | 25 | 38 | 48 | 58 | 73 | 83 | 89 |

### COMPARATIVE EXAMPLE-2

### Normal blending process

### Fesoterodine tablet composition as described in Example-1 was repeated, using the following steps:

1. Fesoterodine hydrogen fumarate., pregelatinized starch, citric acid monohydrate, hydroxypropyl methylcellulose K100 CR, hydroxypropyl methylcellulose K100M CR, hydroxypropyl methylcellulose K4M and colloidal silicon dioxide were sifted through a mesh #40 sieve and blended for 15 minutes.
2. glyceryl behenate was sifted through a mesh #60 sieve.
3. blend of step 1 was lubricated with the glyceryl behenate of step 2.
4. lubricated blend of step 3 was directly compressed into tablets.
5. tablets of step 4 were film coated using an Opadry® dispersion.

The film coated tablets obtained in Example 1 and Comparative Example 2 were evaluated for stability over a period of 1 month at 40°C and 75% relative humidity for the presence of impurities using HPLC, initially and after storage for one month. The results of this measurement were listed as percentage w/w of 2-((R) -3- (diisopropylamino)-1-phenylpropyl)-4-(hydroxymethyl)phenol impurity and total impurities in Table 3.

**Table 3. Results of stability evaluation of fesoterodine film coated tablets**

| Blending process | Initial | | 40°C/75% RH, 1month | |
|---|---|---|---|---|
| | H-FTFRC-01* | Total Impurities | H-FTFRC-01* | Total Impurities |
| Example-1 (Pre-lubrication of excipients with glyceryl behenate) | 0.319 | 0.648 | 1.111 | 1.424 |
| Comparative Example-2 (Normal blending process) | 0.547 | 0.902 | 1.789 | 2.566 |

| | | | | |
|---|---|---|---|---|
| H-FTFRC-01*: 2-((R)-3-(diisopropylamino)-1-phenylpropyl)-4-(hydroxymethyl)phenol. | | | | |

As can be seen from Table 3, Inventive Example-1 has reduced levels of H-FTFRC-01 impurity and total impurities as compared to Comparative Example-2, initially and after storage for one month at 40°C and 75% relative humidity.

Based on the results presented in Table 3, there appears to be a correlation between the formation of impurities and order of mixing drug and excipients in blending process. The formulation of Example-1 involves pre-lubrication of excipients which reduces the interaction between drug and excipients. Initially and after storage for one month, Example-1 had significantly lower levels of 2-((R) -3- (diisopropylamino)-1-phenylpropyl)-4-(hydroxymethyl)phenol impurity and total impurities. In contrast, the formulation of Comparative Example-2 prepared by a conventional blending process, had significantly higher level of 2-((R) -3- (diisopropylamino)-1-phenylpropyl)-4-(hydroxymethyl)phenol impurity and total impurities initially and after storage when compared to formulation Example-1. Thus, the data in Table 3 indicates that the stability of dosage forms of fesoterodine fumarate can be improved based on order/sequence of mixing drug and excipients.

### EXAMPLE 3-5

**Table 4.Fesoterodine tablet compositions prepared by granulation process:**

| Ingredients | Example-3 mg/Tablet (Dry granulation) | Example-4 mg/Tablet (Wet granulation) | Example-5 mg/Tablet (Wet granulation) |
|---|---|---|---|
| Intra-granular ingredients | | | |
| Fesoterodine fumarate | 8.00 | 8.00 | 8.00 |
| Pregelatinized starch | 147.44 | 147.44 | 147.44 |
| Hydroxypropyl methylcellulose K100 CR | 72.00 | 72.00 | 72.00 |
| Hydroxypropyl methylcellulose K100M CR | 48.00 | 48.00 | 48.00 |
| Hydroxypropyl methylcellulose K4M | 24.00 | 24.00 | 24.00 |
| Citric acid monohydrate | 0.66 | 0.66 | 0.66 |
| Colloidal silicon dioxide | 9.90 | 9.90 | 9.90 |
| Glyceryl behenate | 5.00 | 5.00 | 5.00 |
| | | | |
| Granulation | | | |
| Purified water | | q.s. | |
| Isopropyl alcohol | | | q.s. |
| Extragranular ingredients | | | |
| Glyceryl behenate | 5.00 | 5.00 | 5.00 |
| | | | |
| Core tablet weight | 320.0 | 320.0 | 320.0 |
| | | | |
| Film coating | | | |
| Opadry® blue | 10.00 | 10.00 | 10.00 |
| Film coated tablet weight | 330.00 | 330.00 | 330.00 |

### Manufacturing process for Example 3-5:

1. Pregelatinized starch, citric acid monohydrate, hydroxypropyl methylcellulose K100 CR, hydroxypropyl methylcellulose K100M CR, hydroxypropyl methylcellulose K4M and colloidal silicon dioxide were sifted with a half quantity of glyceryl behenate through a mesh #40 sieve and blended for 15 minutes.
2. 10% of the blend of step 1 and fesoterodine fumarate were sifted together through a mesh #40 sieve.
3. sifted materials of step 1 and 2 were blended for 10 minutes.
4. blend of step 3 was slugged/compacted or wet granulated using purified water or isopropyl alcohol, followed by milling and sifting to provide the desired granules.
5. remaining half quantity of glyceryl behenate was sifted through a mesh #60 sieve.
6. granules of step 4 were lubricated with glyceryl behenate of step 5.
7. lubricated blend of step 6 was compressed into tablets.
8. tablets of step 7 were film coated using an Opadry® dispersion.

The tablets obtained in Examples 3 to 5 were subjected to a stability evaluation at 40°C and 75% relative humidity. The tablets were evaluated for the presence of 2-((R) -3-(diisopropylamino)-1-phenylpropyl)-4-(hydroxymethyl)phenol impurity as well as total impurities using HPLC. The results are shown in Table 5.

**Table 5. Results of stability evaluation of fesoterodine tablets**

| Process | H-FTFRC-01* impurity | Total impurities |
|---|---|---|
| Example-1 Direct compression | 0.319 | 0.648 |
| Example-3 Dry granulation | 0.66 | 1.02 |
| Example-4 Wet granulation (with aqueous solvent) | 1.470 | 1.839 |
| Example-5 Wet granulation (with isopropyl alcohol) | 6.119 | 7.229 |

| | | |
|---|---|---|
| H-FTFRC-01*: 2-((R) -3- (diisopropylamino)-1-phenylpropyl)-4-(hydroxymethyl) phenol. | | |

As can be seen from Table 5, Example-1 has reduced amounts of 2-((R) -3-(diisopropylamino)-1-phenylpropyl)-4-(hydroxymethyl)phenol impurity and total impurities at 40°C and 75% relative humidity, as compared to Examples 3, 4 and 5.

Based on the results presented in Table 5, there appears to be a correlation between the formation of impurities and process for the preparation of composition in addition to the excipients used. The formulation of Example-1 prepared by a direct compression process had significantly lower levels of impurities relative to the formulation of Example 3 (dry granulation process) and Examples 4 and 5 (wet granulation process using purified water and isopropyl alcohol respectively). Thus, the data in Table 5 indicates that fesoterodine fumarate compositions prepared by direct compression process were more stable as compared to compositions with dry granulation process and/or wet granulation process.

### EXAMPLE 6

### Fesoterodine tablet compositions prepared by direct compression process:

| Ingredient | mg/Tablet |
|---|---|
| Fesoterodine fumarate | 8.00 |
| Pregelatinized starch | 116.10 |
| Hydroxypropyl methylcellulose K 100 CR | 72.00 |
| Hydroxypropyl methylcellulose K 100M CR | 48.00 |
| Hydroxypropyl methylcellulose K 4M CR | 24.00 |
| Hydroxypropyl cellulose | 32.00 |
| Colloidal silicon dioxide | 9.90 |
| Glyceryl behenate | 10.00 |
| Core tablet weight | 320.00 |
| | |
| Film coating | |
| Opadry® blue | 10.00 |
| Coated tablet weight | 330.00 |

### Manufacturing process:

1. Pregelatinized starch, hydroxypropyl methylcellulose K 100 CR, hydroxypropyl methylcellulose K 100M CR, hydroxypropyl methylcellulose K4M CR, hydroxypropyl cellulose, colloidal silicon dioxide were sifted with a half quantity of glyceryl behenate through a mesh #40 sieve and blended for 15 minutes.
2. 10% of the blend of step 1 and fesoterodine fumarate were sifted together through a mesh #40 sieve.
3. sifted materials of step 1 and 2 were blended for 10 minutes.
4. remaining half quantity of glyceryl behenate was sifted through a mesh #60 sieve.
5. blend of step 3 was lubricated with the glyceryl behenate of step 4.
6. lubricated blend of step 5 was directly compressed into tablets.
7. tablets of step 6 were film coated using an Opadry® dispersion.

### EXAMPLE 7

### Fesoterodine tablet compositions prepared by direct compression process:

| Ingredient | mg/Tablet |
|---|---|
| Fesoterodine fumarate | 8.00 |
| Hydroxypropyl methylcellulose K 100 CR | 72.00 |
| Hydroxypropyl methylcellulose K 100M CR | 48.00 |
| Hydroxypropyl methylcellulose K4M CR | 24.00 |
| Crospovidone | 149.50 |
| Colloidal silicon dioxide | 8.50 |
| Glyceryl behenate | 10.00 |
| | |
| Core tablet weight | 320.00 |
| | |
| Film coasting | |
| Opadry® blue | 10.00 |
| Coated tablet weight | 330.00 |

### Manufacturing process:

1. Hydroxypropyl methylcellulose K 100 CR, hydroxypropyl methylcellulose K 100M CR, hydroxypropyl methylcellulose K4M CR, crospovidone, colloidal silicon dioxide and half quantity of glyceryl behenate were sifted together through mesh #40 and blended for 15 min.
2. 10% blend of step 1 and fesoterodine fumarate were sifted together through mesh #40.
3. sifted materials of step 1 and 2 were blended for 10 minutes.
4. remaining half quantity of glyceryl behenate was sifted through mesh #60.
5. blend of step 3 was lubricated with glyceryl behenate of step 4.
6. lubricated blend of step 5 was compressed into tablets.
7. tablets of step 6 were film coated using an Opadry® dispersion.

## Claims

1. A stable pharmaceutical composition comprising fesoterodine fumarate, glyceryl behenate and a mixture of citric acid and pre-gelatinized starch as stabilizer.

2. The stable pharmaceutical composition of claim 1 in the form of a tablet comprising i) fesoterodine fumarate in an amount of 1% to 5% by weight, ii) glyceryl behenate in an amount of 1% to 8% by weight, iii) pregelatinized starch in an amount of 30% to 50% by weight and iv) a stabilizer in an amount of 0.1% to 10% by weight based on total weight of the composition.

3. The stable pharmaceutical composition of claim 1 and 2, wherein the composition is an extended-release composition.

4. The stable pharmaceutical composition of claim 3, further comprising hydroxypropyl methylcellulose as a controlled-release agent, wherein the hydroxypropyl methylcellulose has a viscosity of 100 cps to 1,00,000 cps.

5. The stable pharmaceutical composition of claim 1 and 2, wherein the composition has a 2-((R)-3-(diisopropylamino)-1-phenylpropyl)-4-(hydroxymethyl)phenol impurity in an amount of less than 2% by weight after storage for one month at 40°C and 75%RH.

6. The stable pharmaceutical composition of claim 1 to 5, further comprising a diluent, a binder, a disintegrant, a lubricant and a glidant, or a combination thereof.

7. The stable pharmaceutical tablet composition of claim 2, further comprising a film coating.

8. The stable pharmaceutical tablet composition of claim 2, wherein the tablet is prepared by direct compression.

9. The stable pharmaceutical composition of claim 1, wherein the composition is a controlled release tablet, comprising pregelatinized starch as a diluent, and hydroxypropyl methylcellulose as controlled-release agent.

10. The stable pharmaceutical tablet composition according to any of the preceding claims, wherein the composition is free from added sugar alcohols.

11. A process for the preparation of pharmaceutical tablet composition of fesoterodine comprising:
(i) sifting and blending one or more pharmaceutically acceptable excipients with glyceryl behenate to form a dry mixture;
(ii) sifting and blending dry mixture of step (i) with fesoterodine;
(iii) optionally lubricating the blend of step (ii); and
(iv) directly compressing the blend of step (ii) or (iii) into tablets.

12. Composition as defined in any of claims 1 to 10 for use in the treatment of overactive bladder with symptoms of urge urinary incontinence, urgency, and frequency.

## Patentansprüche

1. Eine stabile pharmazeutische Zusammensetzung umfassend Fesoterodinfumarat, Glycerylbehenat und eine Mischung aus Zitronensäure und vorverkleisterter Stärke als Stabilisator.

2. Die stabile pharmazeutische Zusammensetzung des Anspruchs 1 in Form einer Tablette umfassend i) Fesoterodinfumarat in einer Menge von 1 Gew.-% bis 5 Gew.-%, ii) Glycerylbehenat in einer Menge von 1 Gew.-% bis 8 Gew.-%, iii) vorverkleisterte Stärke in einer Menge von 30 Gew.-% bis 50 Gew.-% und iv) einen Stabilisator in einer Menge von 0,1 Gew.-% bis 10 Gew.-% bezogen auf das gesamte Gewicht der Zusammensetzung.

3. Die stabile pharmazeutische Zusammensetzung des Anspruchs 1 und 2, wobei die Zusammensetzung eine Zusammensetzung verlängerter Freisetzung ist.

4. Die stabile pharmazeutische Zusammensetzung des Anspruchs 3, weiterhin umfassend Hydroxypropylmethylcellulose als Mittel kontrollierter Freisetzung, wobei die Hydroxypropylmethylcellulose eine Viskosität von 100 cps bis 1,00,000 cps hat.

5. Die stabile pharmazeutische Zusammensetzung des Anspruchs 1 und 2, wobei die Zusammensetzung eine Verunreinigung bestehend aus 2-((R)-3-(Diisopropylamino)-1-phenylpropyl)-4-(hydroxymethyl)phenol in einer Menge von weniger als 2 Gew.-% nach der Lagerung während eines Monats bei 40 °C und 75%RH hat.

6. Die stabile pharmazeutische Zusammensetzung des Anspruchs 1 bis 5, weiterhin umfassend ein Streckmittel, ein Bindemittel, einen Zerfallsbeschleuniger, ein Gleitmittel und ein Fließregulierungsmittel, oder eine Kombination davon.

7. Die stabile pharmazeutische Tablettenzusammensetzung des Anspruchs 2, weiterhin umfassend eine Filmbeschichtung.

8. Die stabile pharmazeutische Tablettenzusammensetzung des Anspruchs 2, wobei die Tablette durch direkte Kompression hergestellt wird.

9. Die stabile pharmazeutische Zusammensetzung des Anspruchs 1, wobei die Zusammensetzung eine Tablette kontrollierter Freisetzung ist, welche vorverkleisterte Stärke als Streckmittel und Hydroxypropylmethylcellulose als Mittel kontrollierter Freisetzung umfasst.

10. Die stabile pharmazeutische Tablettenzusammensetzung nach einem der vorhergehenden Ansprüche, wobei die Zusammensetzung frei von zugegebenen Zuckeralkoholen ist.

11. Ein Verfahren zur Herstellung der pharmazeutischen Tablettenzusammensetzung von Fesoterodin umfassend:
(i) sieben und mischen von einem oder mehreren pharmazeutisch akzeptablen Hilfsstoffen mit Glycerylbehenat, um eine trockene Mischung zu bilden;
(ii) sieben und mischen der trockenen Mischung des Schritts (i) mit Fesoterodin;
(iii) wahlweise schmieren der Mischung des Schritts (ii); und
(iv) direktes komprimieren der Mischung des Schritts (ii) oder (iii).

12. Zusammensetzung wie in einem der Ansprüche 1 bis 10 definiert zur Verwendung in der Behandlung von überaktiver Blase mit Symptomen von Dranginkontinenz, Harndrang und Häufigkeit.

## Revendications

1. Une composition pharmaceutique stable comprenant du fumarate de fésotérodine, du béhénate de glycéryle et un mélange d'acide citrique et d'amidon prégélatinisé comme stabilisateur.

2. La composition pharmaceutique stable de la revendication 1 sous forme d'un comprimé comprenant i) du fumarate de fésotérodine dans une quantité de 1 % à 5 % en poids, ii) du béhénate de glycéryle dans une quantité de 1 % à 8 % en poids, iii) de l'amidon prégélatinisé dans une quantité de 30 % à 50 % en poids et iv) un stabilisateur dans une quantité de 0,1 % à 10 % en poids par rapport au poids total de la composition.

3. La composition pharmaceutique stable de la revendication 1 et 2, dans laquelle la composition est une composition à libération prolongée.

4. La composition pharmaceutique stable de la revendication 3, comprenant en outre de l'hydroxypropylméthylcellulose comme agent à libération contrôlée, dans laquelle l'hydroxypropylcellulose aune viscosité de 100 cps à 1,00,000 cps.

5. La composition pharmaceutique stable de la revendication 1 et 2, dans laquelle la composition a une impureté de 2-((R)-3-(diisopropylamino)-1-phénylpropyl)-4-(hydroxyméthyl)phénol dans une quantité inférieure à 2 % en poids après l'entreposage pendant un mois à 40 °C et 75 % HR.

6. La composition pharmaceutique stable de la revendication 1 à 5, comprenant en outre un diluant, un liant, un agent de désagrégation, un lubrifiant et un agent de glissement, ou une combinaison de ceux-ci.

7. La composition de comprimé pharmaceutique stable de la revendication 2, comprenant en outre un revêtement en film.

8. La composition de comprimé pharmaceutique stable de la revendication 2, dans laquelle le comprimé est préparé par compression directe.

9. La composition pharmaceutique stable de la revendication 1, dans laquelle la composition est un comprimé à libération contrôlée, comprenant de l'amidon prégélatinisé comme diluant, et de l'hydroxypropylméthylcellulose comme agent à libération contrôlée.

10. La composition de comprimé pharmaceutique stable selon l'une quelconque des revendications précédentes, dans laquelle la composition est sans alcools de sucre ajoutés.

11. Un procédé de préparation de composition de comprimé pharmaceutique de fésotérodine comprenant :
(i) tamiser et mélanger un ou plusieurs excipients pharmaceutiquement acceptables avec du béhénate de glycéryle pour former un mélange sec ;
(ii) tamiser et mélanger le mélange sec de l'étape (i) avec de la fésotérodine ;
(iii) facultativement lubrifier le mélange de l'étape (ii); et
(iv) comprimer directement le mélange de l'étape (ii) ou (iii) en formant des comprimés.

12. Composition telle que définie dans l'une quelconque des revendications 1 à 10 pour l'utilisation dans le traitement de l'hyperactivité vésicale avec des symptômes d'incontinence urinaire par impériosité, urgence mictionnelle, et fréquence urinaire.
